Europäisches Patentamt

European Patent Office

Office européen des brevets

(11) Numéro de publication: **0 515 983 A1**

# DEMANDE DE BREVET EUROPEEN

(21) Numéro de dépôt: **92108582.5**

(22) Date de dépôt: **21.05.92**

(51) Int. Cl.5: **A61C 1/00**, A61B 17/32

(30) Priorité: **28.05.91 FR 9106488**

(43) Date de publication de la demande:
**02.12.92 Bulletin 92/49**

(84) Etats contractants désignés:
**CH DE GB IT LI**

(71) Demandeur: **LASAG AG**
**Mittlere Strasse 52**
**CH-3600 Thun(CH)**

(72) Inventeur: **Richerzhagen, Bernold**
**Chemin du Mont-Tendre 10**
**CH-1036 Sullens(CH)**

Inventeur: **Leiglon, Eric**
**Route des Coudres 36**
**CH-1298 Celigny(CH)**
Inventeur: **Tasev, Emile**
**Rue du Centre 74**
**CH-1025 St-Sulpice(CH)**
Inventeur: **Delacrétaz, Guy**
**Chemin Neuf 5**
**CH-1028 Préverenges(CH)**

(74) Mandataire: **de Raemy, Jacques et al**
**ICB Ingénieurs Conseils en Brevets SA**
**Passage Max. Meuron 6**
**CH-2001 Neuchâtel(CH)**

(54) **Dispositif d'ablation de matière, notamment pour la dentisterie.**

(57) Dispositif d'ablation de matière, notamment une pièce à main (1) pour la dentisterie, comprenant des moyens optiques (6, 16, 22, 32) pour la propagation et le guidage jusqu'à une surface de travail d'un faisceau de lumière cohérente (10), ainsi que des moyens de canalisation (24, 30) pour amener un fluide sous pression jusqu'à la tête de travail (5) du dispositif et une buse (20) située dans ladite tête de travail en aval desdits moyens de canalisation et communiquant avec ces derniers pour former un jet (32) de fluide liquide. Le dispositif est caractérisé en ce qu'il comprend une chambre (30) de détente en amont de ladite buse (20) et en ce que l'axe optique (18) du faisceau de lumière cohérente (10) est aligné sur l'axe central du canal de la buse (20), ledit jet (32) de fluide liquide servant de guide d'onde entre ladite tête de travail (5) et la surface de travail.

Fig.1

EP 0 515 983 A1

La présente invention concerne un dispositif d'ablation de matière, et plus particulièrement une pièce à main pour la dentisterie utilisant un faisceau de lumière cohérente. Un tel dispositif comprend aussi des moyens pour former un jet de fluide liquide aboutissant sur la surface traitée par le faisceau de lumière cohérente.

Le travail avec de tels dispositifs nécessite une focalisation correcte du faisceau laser sur la surface à traiter ou, en d'autres termes, une distance relativement constante le long de l'axe optique du faisceau entre cette surface et la sortie du faisceau laser.

Il est connu du brevet US-4,849,859 une pièce à main dentaire utilisant un faisceau laser, ainsi qu'un jet de fluide servant à refroidir et à nettoyer la surface traitée. Le faisceau laser, provenant d'une source laser, est guidé par une fibre optique jusqu'à une tête de travail orientable. Arrivé dans la région de la tête de travail, le faisceau laser sort de l'extrémité sectionnée de la fibre optique et il se dirige vers un miroir sphérique de focalisation solidaire de la tête orientable.

Dans un premier mode de réalisation, le faisceau laser réfléchi par le miroir sphérique sort de la tête de travail de manière libre, la région de focalisation étant située en dehors de la tête de travail. Ce mode de réalisation permet ainsi de travailler avec le faisceau laser sur une surface à traiter sans contact de cette surface avec une pièce appartenant à la tête de travail. Cependant ce mode de réalisation ne permet pas de travailler précisément dans la région de focalisation du faisceau laser, ce qui est un grand inconvénient pour l'efficacité du traitement.

Pour résoudre ce problème, le brevet US-4,849,859 propose un deuxième mode de réalisation dans lequel les moyens de guidage du faisceau laser comprennent sur la fin du chemin optique une sonde (ou fibre optique souple) conique de contact.

Dans ce cas, la focalisation correcte est obtenue en mettant l'extrémité de la sonde directement en contact avec la surface à traiter. Celle-ci est donc encombrée pour la sonde. Ensuite, il est à remarquer que cette solution présente le désavantage du contact physique solide-solide entre la tête de travail et la surface traitée par l'intermédiaire de la sonde conique de contact. Ce contact physique limite sérieusement la puissance du faisceau laser avec laquelle on peut travailler sans endommager l'extrémité de la sonde. Le travail sur des matériaux durs comme les diélectriques solides est problématique, surtout lorsqu'un plasma de surface est engendré par le faisceau laser pendant le traitement. De plus, le jet de fluide, provenant d'un canal situé en amont de la tête de travail, se propage presque toujours dans une direction n'aboutissant pas exactement sur la surface traitée, étant donné que la tête de travail est orientable.

Il est aussi connu de la demande de brevet FR-2,640,537 une pièce à main dentaire similaire au deuxième mode de réalisation du brevet US-4,849,859 décrit ci-dessus. Ce dispositif est prévu pour fonctionner avec un laser du type Nd:YAG pour traiter entre autre la carie dentaire. La tête de travail de ce dispositif est agencée pour recevoir un instrument d'application interchangeable, constitué par une fibre optique souple. Le faisceau laser est focalisé par un miroir sphérique sur l'extrémité de cette fibre optique, laquelle se trouve en contact avec la région traitée lors du traitement.

A nouveau, un contact physique solide-solide entre la tête de travail et la surface traitée est prévu pour assurer la focalisation du faisceau laser sur la surface à traiter. A cet inconvénient majeur s'ajoute le désavantage de la distance fixe qui doit être maintenue entre la tête de travail et la surface à traiter, ce qui limite considérablement la liberté de manoeuvre durant le traitement.

Il est encore connu du brevet US-4,826,431 une pièce à main dentaire utilisant un faisceau laser, ainsi qu'un jet de fluide. La tête de cette pièce à main est agencée de telle manière que le faisceau laser soit conduit au moyen d'une fibre optique jusqu'à proximité de la surface traitée. A côté de cette fibre optique et parallèle à celle-ci, un conduit pour fluide sert à canaliser un fluide jusqu'à la surface traitée. A l'extrémité de la fibre optique conduisant le faisceau laser jusqu'à la surface traitée est prévu un condenseur optique pour focaliser l'énergie lumineuse du faisceau laser sur cette surface.

Cette pièce à main dentaire présente à nouveau le désavantage de devoir être maintenue à une distance fixe de la surface à traiter pour permettre une focalisation correcte. De plus, la surface à traiter est encombrée par l'extrémité du conduit contenant la fibre optique et le conduit pour le fluide.

Afin de maintenir la distance fixe et prédéterminée entre l'extrémité de la fibre optique et la surface traitée, sans pour autant être en contact avec cette surface, il est connu du brevet US-4,503,853 d'utiliser un cylindre d'application qui forme une entretoise entre la sortie de la lumière cohérente et la surface à traiter. Cependant, cette solution enlève toute liberté de manoeuvre au praticien et en plus elle gêne l'observation de la surface traitée par celui-ci.

La présente invention a pour but de pallier les inconvénients de ces dispositifs connus en fournissant une pièce à main, utilisant un faisceau de lumière cohérente et un jet de fluide liquide servant notamment à refroidir et à nettoyer la surface traitée, qui assure une liberté de mouvement entre la tête de travail et la surface traitée selon la direction du faisceau de lumière cohérente, tout en garantissant un bon rendement quelle que soit la distance relative entre la tête de travail et la surface traitée pour toute une plage de valeurs possibles, sans nécessiter de mécanismes délicats et compliqués.

La présente invention a donc pour objet un dispositif d'ablation de matière, notamment une pièce à main pour la dentisterie, comprenant :

- un carter définissant un corps et une tête de travail;
- des moyens optiques pour la propagation et le guidage jusqu'à une surface de travail d'un faisceau de lumière cohérente, ces moyens optiques définissant un axe optique et destinés à être branchés sur une source de lumière cohérente;
- des moyens de canalisation pour amener un fluide sous pression jusqu'à ladite tête de travail et destinés à être branchés sur une source de fluide liquide sous pression;
- une buse située dans ladite tête de travail en aval desdits moyens de canalisation et communiquant avec ces derniers pour former un jet de fluide liquide;
- ledit dispositif étant caractérisé en ce que le canal de ladite buse est sensiblement aligné sur ledit axe optique, lesdits moyens de canalisation comportant une chambre comprenant au moins un volume situé immédiatement en amont de ladite buse et destiné à recevoir ledit fluide liquide sous pression, ledit volume étant traversé par ledit faisceau de lumière cohérente avant que celui-ci pénètre dans le canal de ladite buse, ledit fluide liquide amené sous pression dans ledit volume et dans le canal de ladite buse, ainsi que dans ledit jet de fluide liquide engendré par cette dernière servant de moyens optiques de propagation et de guidage dudit faisceau de lumière cohérente.

Il résulte de ces caractéristiques un dispositif d'ablation de matière capable de fournir un jet de fluide liquide parfaitement laminaire sur une distance de l'ordre du centimètre, ce qui permet de guider directement la lumière cohérente sur la surface à traiter, sans qu'il soit nécessaire de respecter une distance précise entre la tête de travail et cette surface et sans que celle-ci soit cachée à l'opérateur pendant le travail. De plus, le dispositif assure une focalisation du faisceau de lumière cohérente sur la surface traitée sans aucun contact physique solide-solide.

L'écoulement parfaitement laminaire du fluide liquide dans le canal de la buse, ainsi que dans le jet engendré par cette dernière résulte premièrement du volume situé directement en amont de la buse et destiné à recevoir uniquement le fluide liquide sous pression. Ce volume, ainsi que le canal de la buse sont donc entièrement libres et destinés à ne contenir que le fluide sous pression servant à engendrer le jet de fluide liquide.

Le volume se trouvant directement en amont de la buse est compris dans une chambre servant de chambre de détente. Cette dernière assure une quasi stagnation du fluide qui est amené sous pression.

Deuxièmement, l'écoulement parfaitement laminaire du jet de fluide liquide résulte de la sélection d'une buse appropriée. On remarque ainsi que la chambre prévue en amont de la buse et contenant le volume libre précédant l'entrée du canal de cette buse, ainsi que la buse avantageusement sélectionnée assurent un écoulement laminaire sans turbulence ni bulles microscopiques dans le volume libre, dans le canal de la buse et dans le jet laminaire de fluide liquide engendré par cette dernière.

Comme le dispositif est conçu de manière que l'axe optique du faisceau de lumière cohérente soit aligné sur le canal de la buse, la lumière cohérente qui pénètre dans ce canal et qui en ressort avec un angle de divergence permettant d'obtenir une réflexion totale à l'interface fluide liquide - air. Ainsi, cette lumière cohérente ne peut se propager qu'à l'intérieur du jet laminaire de fluide liquide. Le jet laminaire de fluide liquide sert donc de guide optique pour la lumière cohérente jusqu'à proximité de la surface traitée par le faisceau de lumière cohérente.

Ce résultat de l'invention répond ainsi au but essentiel de l'invention, à savoir la liberté de manoeuvre dans le positionnement de la tête de la pièce à main relativement à la surface traitée. Il est en effet possible de faire varier la distance relative entre la tête de la pièce à main et la surface traitée sans nuire à l'efficacité du traitement, la longueur du guide d'onde fluide pouvant varier d'une valeur quasi nulle à une valeur d'au moins l'ordre du centimètre.

Le volume libre précédant la buse est lui-même précédé par un autre moyen de propagation et de guidage du faisceau de lumière cohérente. Afin d'obtenir un très bon rendement dans le couplage du faisceau de lumière cohérente avec le jet de fluide liquide, il est prévu, dans le mode de réalisation préféré, une optique de focalisation qui focalise le faisceau de lumière cohérente de manière que la tache focale soit à l'intérieur du canal de la buse. Dans ce cas-ci, la dernière lentille de focalisation est incorporée dans une paroi de la chambre qui est opposée à la paroi comprenant la buse; elle définit donc une fenêtre transparente dans une paroi de la chambre. Ainsi, le volume libre dans lequel se propage le faisceau de lumière cohérente est défini sensiblement par la chambre elle-même.

Dans un autre mode de réalisation, le moyen de propagation et de guidage en amont du volume précédant la buse consiste en une fibre optique dont l'extrémité est en forme de dioptre poli. La fibre est positionnée dans la chambre de manière que l'essentiel du faisceau de lumière cohérente entre dans le canal de la buse, afin d'assurer un bon rendement dans le couplage optique avec le jet de fluide liquide, mais sans nuire à l'écoulement parfaitement laminaire du fluide liquide sous pression se dirigeant vers l'entrée du canal de la buse et se trouvant à l'intérieur du volume libre.

Le dispositif selon l'invention garantit un chemin optique de très bonne qualité pour le faisceau de lumière cohérente se propageant dans le fluide liquide à travers le volume libre susmentionné et le canal de la buse, puis à l'intérieur du jet laminaire. Ainsi, la qualité du chemin optique dans le fluide liquide entre la dernière lentille de focalisation ou l'extrémité de la fibre optique et la région avoisinant la surface traitée permet au dispositif selon l'invention de coupler de très hautes densités de puissance de lumière cohérente, à la limite du claquage optique, dans le jet de fluide liquide. Ceci est très avantageux et même essentiel pour obtenir une ablation de matière sur un matériau, comme par exemple l'émail ou la dentine, avec un bon rendement.

Un autre avantage du dispositif selon l'invention vient du fait qu'aucune pièce en matière solide ne vient s'appliquer sur la surface traitée ou sur la région avoisinante.

Il est aussi à remarquer que le jet de fluide qui guide le faisceau de lumière cohérente jusqu'à la surface traitée peut servir conjointement de moyens de refroidissement, mais aussi de nettoyage ou encore de moyens de désinfection de cette zone de traitement dans le cas de la dentisterie, pour autant que le fluide liquide sélectionné possède les qualités optiques requises conjointement à l'une de ces propriétés spécifiques.

Il est aussi possible d'utiliser un liquide coloré, afin de permettre la visualisation du jet de fluide liquide et par conséquent du faisceau de lumière cohérente utilisé.

L'invention sera mieux comprise à l'aide de la description qui va suivre et des dessins qui l'illustrent à titre d'exemple. Sur ces dessins :

- la figure 1 représente schématiquement une vue en coupe longitudinale d'une pièce à main avec une optique de focalisation selon l'invention;
- la figure 2 montre schématiquement et à grande échelle une partie de la tête de la pièce à main selon l'invention pour le mode de réalisation de la figure 1;
- les figures 3 à 5 représentent schématiquement trois variantes d'un mode de réalisation sans optique de focalisation de la pièce à main selon l'invention.

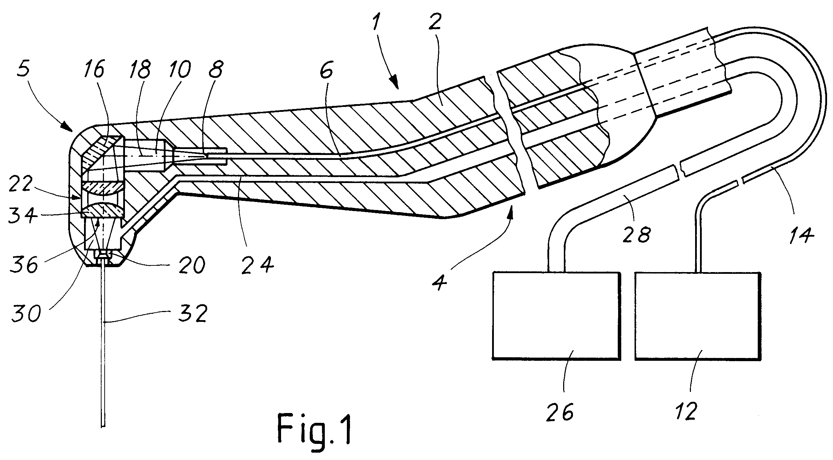

En se référant à la figure 1, on décrira ci-après un premier mode de réalisation selon l'invention, appliquée spécifiquement à une pièce à main de dentisterie, cette application n'étant nullement limitative. En effet, l'invention couvre aussi d'autres outillages utilisables par exemple dans l'industrie et destinés à assurer l'ablation de matière.

Cette pièce à main 1 comprend un carter 2 comprenant un corps de saisie 4 ainsi qu'une tête de travail 5. A l'intérieur du carter 2 est placée une fibre optique 6 servant de moyens pour la propagation et le guidage, jusqu'à l'extrémité 8 de cette fibre optique, d'un faisceau de lumière cohérente 10. La fibre optique 6 est destinée à être reliée à une source 12 de lumière cohérente par des moyens flexibles 14 de propagation et de guidage du faisceau de lumière cohérente 10. De manière préférée, les moyens flexibles 14 sont formés par le prolongement de la fibre optique 6, ce qui évite une connexion entre deux moyens de propagation et de guidage. La source 12 de lumière cohérente consiste par exemple en un laser du type Nd:YAG alimenté en mode pulsé et fonctionnant en multimode ou en mode fondamental TEM$_{oo}$. Naturellement, d'autres types de laser peuvent être utilisés.

De manière avantageuse, la fibre optique 6 est effilée dans le sens de la propagation de la lumière. Un profil effilé permet de condenser le faisceau de lumière cohérente 10, de telle manière que sa section soit suffisamment petite à l'extrémité 8 de la fibre optique 6 située dans la région de la tête de travail 5.

Le faisceau de lumière cohérente 10 provenant de la source 12 de lumière cohérente est donc guidé jusqu'à ladite extrémité 8 de la fibre optique 6, de laquelle il sort avec une certaine ouverture définissant un angle de divergence de ce faisceau de lumière cohérente 10.

Une fois sorti de la fibre optique 6, le faisceau de lumière cohérente 10 devenu divergent se propage dans l'air ou dans le vide jusqu'à un miroir directionnel 16 situé à l'intérieur de la tête de travail 5. Ce miroir directionnel 16 permet de changer la direction de propagation du faisceau de lumière cohérente 10. Il est ajusté de telle manière que l'axe optique 18 du faisceau de lumière cohérente 10 soit exactement aligné sur le canal d'une buse 20 située en aval de ce miroir directionnel 16. Afin de refocaliser le faisceau de lumière cohérente 10 devenu divergent à la sortie de l'extrémité 8 de la fibre optique 6, une optique de focalisation 22 est prévue entre le miroir directionnel 16 et la buse 20. Cette optique de focalisation 22 sert donc à refocaliser le faisceau de lumière cohérente 10 pour qu'il entre dans le canal de la buse 20.

On notera que le miroir directionnel 16 n'est pas forcément plan, mais qu'il peut aussi être par exemple sphérique afin de participer à la refocalisation du faisceau 10. De manière générale, ce miroir peut être remplacé par d'autres éléments réalisant la même fonction, par exemple un prisme. On notera encore que, dans d'autres modes de réalisation non-représentés, l'optique de focalisation 22 peut être placée en amont du miroir directionnel 16 ou partiellement en amont et partiellement en aval de ce dernier. Finalement, un autre mode de réalisation non-représenté comprend une optique de focalisation sans miroir directionnel, l'extrémité 8 de la fibre optique 6 étant alignée sur le canal de la buse.

La pièce à main 1 comprend aussi à l'intérieur du carter 2 un conduit 24 d'amenée d'un fluide liquide sous pression. Ce conduit 24 est destiné à être relié à une source 26 de fluide liquide sous pression par le moyen d'un conduit flexible 28. Le conduit 24 d'amenée d'un fluide liquide sous pression s'étend jusque dans la tête de travail 5 et aboutit dans une chambre 30 de détente précédant la buse 20. On remarquera que le conduit 24 peut sans autre se séparer en plusieurs conduits de section inférieure avant de déboucher dans la chambre 30. Cependant, il est prévu que la section totale du ou des conduit(s) d'amenée du fluide liquide soit dimensionnée de manière à assurer une quasi-stagnation du fluide liquide dans la chambre 30.

La chambre 30 assure donc une quasi stagnation du fluide liquide sous pression, avant que celui-ci entre dans le canal de la buse 20 pour former un jet laminaire 32 de fluide liquide. La chambre 30 définit un volume situé entre la buse 20 et la dernière lentille de focalisation 34, cette dernière définissant une fenêtre transparente 36 entre l'optique de focalisation 22 et la chambre 30 pour permettre au faisceau de lumière cohérente 10 de pénétrer dans celle-ci. Ainsi, le faisceau de lumière cohérente 10 sortant de la dernière lentille de focalisation 34 se propage directement dans le fluide liquide sous pression se trouvant à l'intérieur de la chambre de détente 30. A la sortie de l'optique de focalisation 22, l'interface est donc un interface lentille-fluide liquide. Le faisceau 10 se propage ainsi dans le fluide liquide sous pression jusqu'à l'entrée du canal de la buse 20 pour être ensuite couplé avec le jet laminaire 32 de fluide liquide. Ce jet laminaire 32 sert alors de guide optique pour le faisceau de lumière cohérente 10.

On notera que, dans un autre mode de réalisation non-représenté, la fenêtre transparente 36 dans la paroi de la chambre 30 peut être formée par une simple lame en matière solide transparente et rien n'empêche de placer le miroir directionnel ou/et certains moyens de focalisation à l'intérieur de la chambre 30, ce qui permet par exemple d'agencer la fenêtre transparente 36 dans une des parois latérales de la chambre 30.

On remarquera aussi que la pièce à main 1 représentée dans cette figure 1 est très schématique. Par exemple, les dimensions de l'optique de focalisation 22 relativement aux dimensions de la chambre 30 de détente ou de la buse 20 ne sont pas à la même échelle. Ensuite, la représentation des divers éléments optiques 16, 22 schématisent seulement les fonctions nécessaires au mode de réalisation décrit, la confection et l'agencement de ces divers éléments optiques étant connus du spécialiste en optique laser.

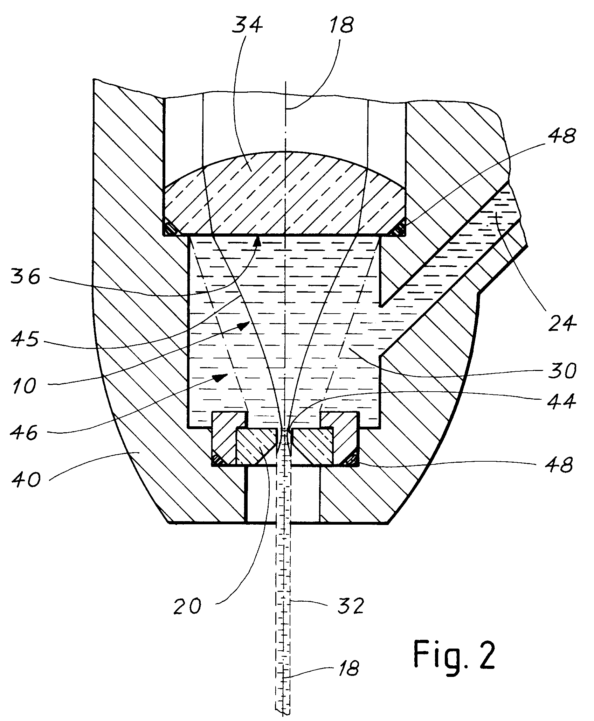

En se référant à la figure 2, on décrira ci-après plus précisément le chemin optique du faisceau de lumière cohérente se propageant dans le fluide liquide.

Cette figure 2 représente schématiquement une partie de la tête de travail 5 décrite à propos de la figure 1. Cette tête de travail comprend un carter 40 à l'intérieur duquel se trouve la dernière lentille 34 de l'optique de focalisation 22 et la buse 20, la chambre 30 de détente étant située entre la dernière lentille 34 et la buse 20. La dernière lentille 34 de l'optique de focalisation 22 définit ici l'axe optique 18 du faisceau de lumière cohérente 10, cet axe optique 18 étant exactement aligné sur l'axe du canal 44 de forme cylindrique appartenant à la buse 20.

Le fluide liquide sous pression amené par le conduit 24 arrive dans la chambre 30 de détente dans laquelle il est maintenu dans un état quasi stationnaire. Depuis la chambre 30, ce fluide liquide s'échappe à travers le canal 44 de la buse 20, formant ainsi un jet laminaire 32 de fluide liquide.

La buse 20 doit présenter au moins deux caractéristiques essentielles pour le bon fonctionnement de cette pièce à main dentaire. La première consiste en un dimensionnement approprié du canal permettant d'engendrer un jet laminaire de fluide liquide de haute qualité.

A titre d'exemple uniquement, le diamètre du canal 44 de forme cylindrique de la buse 20 peut varier entre 10 et 500 $\mu$m, alors que sa longueur varie entre 50 $\mu$m et 1 mm.

La deuxième caractéristique essentielle de cette buse 20 réside dans le matériau utilisé pour sa construction. De manière préférée, ce matériau doit être dans une matière dure qui absorbe très faiblement l'énergie lumineuse. Des matériaux transparents comme le saphir ou le quartz répondent correctement à ces deux caractéristiques. Des buses présentant ces deux caractéristiques essentielles sont fabriquées par exemple chez Comadur S.A., division Thoune, Bernstrasse 11, Ch-3605 Thun, Suisse.

Afin d'éviter des pertes d'énergie lumineuse trop importantes au niveau de la buse 20 et surtout pour limiter les risques de turbulences, un canal d'une longueur relativement petite est avantageux et il est de première importance que ce canal soit parfaitement aligné sur l'axe optique 18 du faisceau de lumière cohérente 10. Afin d'obtenir le meilleur couplage du faisceau de lumière cohérente 10 dans le jet de fluide liquide 32, le faisceau de lumière cohérente 10 est focalisé de telle manière que la tache focale soit située à l'intérieur du canal 44 de la buse 20 et que l'enveloppe 45 du faisceau de lumière cohérente 10 ne touche pas la paroi du canal 44 de la buse 20. Il est donc prévu une optique de focalisation qui garantit une telle propriété.

Comme l'angle maximal en-dessous duquel il y a réflexion totale des rayons lumineux du faisceau de lumière cohérente 10 dans un jet laminaire d'eau est d'environ 50°, l'agencement des divers moyens optiques utilisés est choisi de telle manière que l'angle d'ouverture du faisceau 10, divergeant à nouveau après la tache focale, ne dépasse pas cette valeur limite d'environ 50° dans le cas où le fluide liquide est de l'eau. Cependant, quel que soit le fluide liquide utilisé, les divers moyens optiques sont agencés pour assurer une réflexion totale à l'intérieur du jet laminaire 32 de fluide liquide.

Afin de garantir un chemin optique de première qualité pour le faisceau de lumière cohérente 10 se propageant dans le fluide liquide sous pression, un volume libre 46 situé à l'intérieur de la chambre de détente 30 comprend au moins la totalité du volume traversé par le faisceau de lumière cohérente 10 circulant entre la dernière lentille de focalisation 34 et l'entrée du canal 44 de la buse 20. Ensuite, le faisceau de lumière cohérente 10 convergeant vers l'entrée du canal 44 pénètre dans celui-ci sans traverser d'interfaces, ce canal 44 étant lui-même entièrement libre.

Le jet laminaire de fluide liquide 32 est engendré par le fluide liquide se trouvant sous pression à l'intérieur de la chambre 30 de détente. A titre d'exemple uniquement, la pression du fluide liquide se trouvant à l'intérieur de cette chambre 30 a une valeur comprise entre 0,5 et 100 bars environ. Le fluide liquide se trouvant dans le volume libre 46 est donc lui-même sous pression, ce qui augmente l'homogénéité du fluide liquide et donc la qualité du chemin optique pour le faisceau de lumière cohérente 10 à l'intérieur de ce volume libre 46.

Le jet laminaire 32 de fluide liquide, engendré par les divers moyens appropriés décrits ci-avant, est parfaitement laminaire depuis l'entrée du canal 24 de la buse 20 jusqu'à une distance d'au moins l'ordre du centimètre. La distance sur laquelle le jet laminaire de fluide liquide 32 présente un écoulement laminaire de haute qualité dépend naturellement de la pression du fluide liquide à l'intérieur de la chambre 30 de détente. Ainsi, en faisant varier la pression dans cette chambre 30, il est possible de faire varier la distance sur laquelle le jet laminaire présente un écoulement laminaire de qualité suffisante, ce qui définit une liberté de manoeuvre le long de l'axe optique 42 sur une certaine distance pour l'opérateur.

On remarquera encore que la chambre 30 est totalement étanche, l'étanchéité étant assurée, par exemple, par les joints 48 comme schématisé sur cette figure 2.

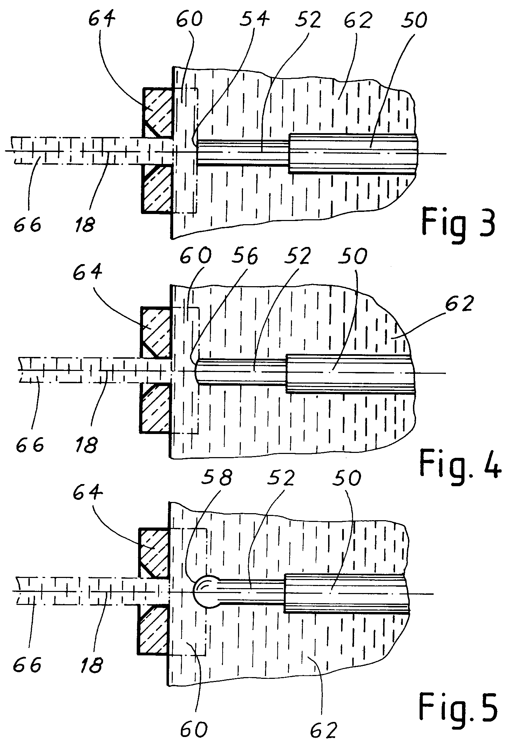

En se référant aux figures 3 à 5, on décrira ci-après trois variantes possibles d'un autre mode de réalisation de la pièce à main selon l'invention.

Dans ce mode de réalisation, l'optique de focalisation est supprimée et une fibre optique 50, dont la dernière partie 52 est dégainée, est alignée sur le canal de la buse 64, cette buse étant située dans la tête de travail 5 de manière similaire à la buse 20 des figures 1 et 2.

La buse 64, tout comme la buse 20 du premier mode de réalisation, ne présente pas forcément la configuration particulière avec un canal cylindrique suivi d'une ouverture tronconique. Toutes autres configurations garantissant les caractéristiques nécessaires du dispositif selon l'invention sont sans autres possibles.

Dans la première variante décrite par la figure 3, cette fibre optique 50 présente une surface d'extrémité 54 plane.

Dans la deuxième variante décrite par la figure 4, l'extrémité 56 de la fibre optique 50 est polie en forme de dioptre convexe. Ceci a pour effet de maintenir le diamètre du faisceau de lumière cohérente sortant de cette fibre sensiblement égal à celui de cette dernière sur une certaine distance avant qu'il ne diverge.

Dans la troisième variante décrite par la figure 5, l'extrémité 58 de la fibre optique 50 est de forme sphérique obtenue par fusion. Cette extrémité 58 assure une certaine refocalisation du faisceau de lumière cohérente sortant de la fibre optique 50.

Dans ces trois variantes, le faisceau de lumière cohérente sortant de la fibre optique 50 par l'extrémité 54, 56 ou 58 traverse le volume libre 60 situé à l'intérieur de la chambre de détente 62 (les parois de cette chambre n'étant pas représentées) avant que ce faisceau de lumière cohérente ne pénètre dans le canal de la buse 64 pour être ensuite couplé avec le jet laminaire de fluide liquide 66 sortant du canal de la buse 64. L'axe optique 18 du faisceau de lumière cohérente est défini dans la région du volume libre 60 par la disposition de la dernière partie 52 de la fibre optique 50. Cette dernière partie étant alignée sur le canal de la buse 64, l'axe optique 18 se confond avec l'axe central du canal de la buse 64.

La disposition de la fibre optique 50 par rapport à l'entrée de la buse 64 est prévue de telle manière que le volume libre 60 soit de dimensions suffisantes pour assurer un écoulement parfaitement laminaire sur tout le chemin optique emprunté par le faisceau de lumière cohérente 10 depuis l'extrémité 54, 56 ou 58 de la fibre optique 50.

Une valeur typique pour le diamètre de la section de la fibre optique 50 sur sa dernière partie 52 est de l'ordre de 100 à 300 $\mu$m; mais d'autres dimensions sont possibles. De manière générale, on choisit un diamètre pour le canal de la buse 64 qui soit au moins égal à celui de la section susmentionnée pour la partie 52 de la fibre optique.

Le dispositif selon l'invention peut être associé à une installation d'ablation de matière comprenant, par exemple, un laser pulsé du type Nd:YAG servant de source 12 de lumière cohérente et engendrant des impulsions lumineuses dont la puissance instantanée atteint des valeurs suffisamment élevées pour que la densité de puissance instantanée du faisceau de lumière cohérente 10 se propageant dans le jet laminaire 32 de fluide liquide présente des pics compris entre $10^7$ et $10^{10}$ W/cm$^2$.

**Revendications**

1. Dispositif d'ablation de matière, notamment pièce à main (1) pour la dentisterie, comprenant :
   - un carter (2) définissant un corps (4) et une tête de travail (5);
   - des moyens optiques (6, 16, 22, 32; 50, 52, 66) pour la propagation et le guidage jusqu'à une surface de travail d'un faisceau de lumière cohérente (10), ces moyens optiques définissant un axe optique (18) et étant destinés à être branchés sur une source (12) de lumière cohérente;
   - des moyens de canalisation (24, 30; 62) pour amener un fluide sous pression jusqu'à ladite tête de travail (5) et destinés à être branchés sur une source (26) de fluide liquide sous pression;
   - une buse (20; 64) située dans ladite tête de travail (5) en aval desdits moyens de canalisation (24, 30; 62) et communiquant avec ces derniers pour former un jet (32) de fluide liquide;
   - ledit dispositif étant caractérisé en ce que le canal (44) de ladite buse (20; 64) est sensiblement aligné sur ledit axe optique (18), lesdits moyens de canalisation comportant une chambre (30; 62) comprenant au moins un volume (46; 60) situé immédiatement en amont de ladite buse (20; 64) et destiné à recevoir ledit fluide liquide sous pression, ledit volume (46; 60) étant traversé par ledit faisceau de lumière cohérente (10) avant que celui-ci pénètre dans le canal (44) de ladite buse (20; 64), ledit fluide liquide amené sous pression dans ledit volume (46; 60) et dans le canal (44) de ladite buse (20; 64), ainsi que ledit jet (32) de fluide liquide engendré par cette dernière servant de moyens optiques de propagation et de guidage dudit faisceau de lumière cohérente (10).

2. Dispositif selon la revendication 1, caractérisée en ce que lesdits moyens optiques de propagation et le guidage comprennent:
   - une fibre optique (6) située à l'intérieur dudit carter (2) essentiellement dans la région dudit corps (4) servant de corps de saisie, une extrémité (8) de cette fibre optique (6) étant située dans la région de ladite tête de travail (5); et
   - des moyens de focalisation (22) dudit faisceau de lumière cohérente (10), situés dans la région de ladite tête de travail (5) en aval de ladite extrêmité (8) de la fibre optique (6) et en amont de ladite buse (20), servant à focaliser ledit faisceau de lumière cohérente (10) de manière que la tache de focalisation soit située à l'intérieur dudit canal (44) de la buse (20).

3. Dispositif selon la revendication 2, caractérisé en ce qu'il comprend au moins un miroir directionnel (16), situé dans ladite tête de travail (5) en aval de ladite extrêmité (8) de la fibre optique (6) et en amont de ladite buse (20), servant à orienter ledit faisceau de lumière cohérente (10) de manière que l'axe optique (18) soit aligné sur ledit canal (44) de ladite buse (20).

4. Dispositif selon la revendication 2 ou 3, caractérisé en ce que lesdits moyens de focalisation (22) comprennent une lentille (34) incorporée dans une paroi de ladite chambre (30) et alignée sur ledit axe optique du faisceau de lumière cohérente, cette lentille (34) définissant une fenêtre transparente (36) dans une paroi de la chambre (30) pour permettre au faisceau de lumière cohérente (10) de pénétrer dans cette dernière.

5. Dispositif selon l'une quelconque des revendications 1 à 4, caractérisé en ce que la section de l'enveloppe (45) du faisceau de lumière cohérente (10) dans la région dudit canal (44) de ladite buse (20) est plus faible que celle de ce canal (44).

6. Dispositif selon l'une quelconque des revendications 1 à 5, caractérisé en ce que la pression du fluide liquide dans la chambre (30) est comprise entre 0,5 et 100 bars.

7. Dispositif selon la revendication 1, caractérisé en ce que lesdits moyens optiques de propagation et de guidage comprennent une fibre optique (50) dont l'extrémité (54, 56, 58) est située directement en amont dudit volume (60) situé dans ladite chambre (62) et précédant directement ladite buse (64), la dernière partie (52) de cette fibre optique (50) étant alignée sur ledit canal de ladite buse (64).

8. Dispositif selon la revendication 7, caractérisé en ce que l'extrémité (56) de ladite fibre optique présente un embout en forme de dioptre convexe poli.

9. Dispositif selon la revendication 7, caractérisé en ce que l'extrémité (58) de ladite fibre optique présente un embout sphérique dioptrique obtenu par fusion.

10. Dispositif selon l'une quelconque des revendications 2 à 9, caractérisé en ce que la section de ladite fibre optique (6; 50) se rétrécit suivant la longueur de cette fibre dans la direction de propagation du faisceau de lumière cohérente (10).

11. Dispositif selon l'une quelconque des revendications 1 à 10, caractérisé en ce que ladite buse (20; 64) est usinée dans un matériau solide transparent absorbant très faiblement l'énergie lumineuse, ledit canal (44) de cette buse étant sensiblement cylindrique.

12. Dispositif selon la revendication 11, caractérisé en ce que ladite buse (20; 64) est en saphir.

13. Dispositif selon la revendication 11, caractérisé en ce que le diamètre dudit canal (44) de ladite buse (20; 64) est compris entre 10 et 1000 $\mu$m.

14. Installation d'ablation de matière comportant un dispositif suivant l'une quelconque des revendications 1 à 13 et une source (12) de lumière cohérente comprenant un laser pulsé du type Nd:YAG engendrant des impulsions lumineuses dont la densité de puissance instantanée dans le jet (32) de fluide liquide présente des pics compris entre $10^7$ W/cm$^2$ et $10^{10}$ W/cm$^2$.

Fig.1

EP 0 515 983 A1

Fig. 2

Fig 3

Fig. 4

Fig.5

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| D,A | FR-A-2 599 961 (KABUSHIKI KAISHA MORITA)<br>* page 15, ligne 3 - ligne 32; figures 1,6,7 *<br>--- | 1,2,7 | A61C1/00<br>A61B17/32 |
| A | EP-A-0 392 951 (LEVY)<br>* abrégé; figure 1 *<br>--- | 1-3,5,14 | |
| A | EP-A-0 346 712 (RAUSIS)<br>* colonne 2, ligne 44 - colonne 3, ligne 44; figure 2 *<br>--- | 1,3 | |
| A | DE-A-3 415 293 (AIHARA)<br>* abrégé; figures 2,4,8 *<br><br>----- | 1-4 | |

**DOMAINES TECHNIQUES RECHERCHES (Int. Cl.5)**

A61C
A61B

Le présent rapport a été établi pour toutes les revendications

| Lieu de la recherche | Date d'achèvement de la recherche | Examinateur |
|---|---|---|
| LA HAYE | 31 AOUT 1992 | KOUSOURETAS I. |

CATEGORIE DES DOCUMENTS CITES

X : particulièrement pertinent à lui seul
Y : particulièrement pertinent en combinaison avec un
autre document de la même catégorie
A : arrière-plan technologique
O : divulgation non-écrite
P : document intercalaire

T : théorie ou principe à la base de l'invention
E : document de brevet antérieur, mais publié à la
date de dépôt ou après cette date
D : cité dans la demande
L : cité pour d'autres raisons
 
& : membre de la même famille, document correspondant

EPO FORM 1503 03.82 (P0402)